# EUROPEAN PATENT SPECIFICATION

(11) **EP 2 520 305 B1**
(45) Date of publication and mention of the grant of the patent: **02.08.2017**
(21) Application number: 09852695.7
(22) Date of filing: 28.12.2009
(51) Int. Cl.: A61K 36/28, A61K 31/353, A61K 31/352, A61K 31/56, A61K 31/365, A61K 31/215, A61K 31/047, A61K 31/19, A61K 31/192, A61K 31/194, A61K 9/00, A61K 9/08, A61K 9/10, A61K 9/14, A61K 9/20, A61K 9/48, A61P 9/12, A61K 127/00, A61K 133/00, A61K 135/00

(54) **PHARMACEUTICAL COMPOSITION INCLUDING SUNFLOWER EXTRACT, PREPARATIVE METHOD AND USE THEREOF**
PHARMAZEUTISCHE ZUSAMMENSETZUNG MIT SONNENBLUMENEXTRAKT, HERSTELLUNGSVERFAHREN UND VERWENDUNG DAVON
COMPOSITION PHARMACEUTIQUE COMPRENANT UN EXTRAIT DE TOURNESOL, PROCÉDÉ DE PRÉPARATION ET D'UTILISATION DE CELLE-CI

(43) Date of publication of application: 07.11.2012
(73) Proprietor: Ling, Peixue, Shandong 250101 (CN)
(72) Inventor: ZHANG, Ling, Jinan Shandong 250101 (CN); SHAO, Chenglei, Jinan Shandong 250101 (CN); JI, Tao, Jinan Shandong 250101 (CN); SHANG, Lixia, Jinan Shandong 250101 (CN); FAN, Zhiping, Jinan Shandong 250101 (CN); LING, Peixue, Jinan Shandong 250101 (CN)
(74) Representative: Potter Clarkson LLP
(86) International application number: PCT/CN2009/001573
(87) International publication number: WO 2011/079410

(56) References cited:
- WO-A1-2008/125433
- CN-A- 1 513 492
- CN-A- 101 473 934
- JP-A- 61 178 908
- GUTIERREZ A B ET AL: "Guaianolides and other constituents of Helianthus microcephalus", PHYTOCHEMISTRY, PERGAMON PRESS, GB, vol. 27, no. 7, 1 January 1988 (1988-01-01), pages 2225-2228, XP026615999, ISSN: 0031-9422, DOI: 10.1016/0031-9422(88)80131-6 [retrieved on 1988-01-01]
- HERZ W ET AL: "ENT PIMARANES ENT KAURANES HELIANGOLIDES AND OTHER CONSTITUENTS OF 3 HELIANTHUS-SPP", PHYTOCHEMISTRY (OXFORD), vol. 23, no. 7, 1984, pages 1453-1460, XP26605966, ISSN: 0031-9422
- ALI A. ALFATAFTA ET AL.: 'Epicuticular terpenoids and an aurone from flowers of Helianthus annuus' PHYTOCHEMISTRY vol. 31, no. 12, 1992, pages 4109 - 4113
- FRANCISCO A. MACIAS ET AL.: 'Bioactive flavonoids from Helianthus annuus cultivars' PHYTOCHEMISTRY vol. 45, no. 4, 1997, pages 683 - 687
- HAI LINA: 'Advance on the study of natural anti-hypertensive active ingredients' JOURNAL OF YUNNAN NORMAL UNIVERSITY vol. 22, no. 4, July 2002, pages 74 - 78
- WANG SHAOYUN ET AL.: 'Advance on the study of nevadensin' JOURNAL OF KAILI UNIVERSITY vol. 26, no. 6, December 2008, pages 47 - 49

## Description

### Field of Invention

This invention relates to a pharmaceutical composition including plant extract, its preparative method and use. Specifically, this invention relates to a pharmaceutical composition including sunflower extract, its preparative method and use. More specifically, this invention relates to a pharmaceutical composition including the extract of leaves, heads or stems of sunflower, its preparative method and use.

### Background of Invention

Essential hypertension is a common and frequently-occurring disease in clinical practice, wherein its etiology is not yet very clear and high blood pressure is the main clinical manifestations for this disease. Although hypertension has a relative independence in classification of diseases, it is an important risk factor for stroke, coronary heart disease, kidney failure and retinopathy and it is a disease as a serious hazard to human health and affecting the quality of life. According to statistics, the prevalence of hypertension is 18.8% in the residents more than 18 years old in China, which increases 31% compared to 1991, now the number of hypertensive patients has been up to 160 million in China. Therefore, how to preventing and treating hypertension has an important meaning. The facts have proved that the intervention for hypertension as a risk factor could reduce the risk of cardiovascular and cerebrovascular diseases, diabetes, cancer and some other chronic diseases simultaneously. Reducing per 3mmHg of diastolic blood pressure of hypertensive patients in average, the risk of stroke decreases by 32% and the risk of coronary heart disease drops by 19%. Therefore, controlling hypertension is an efficient primary prevention measure which could make the medical costs and social burdens reduced efficiently. If calculated by decreasing 3mm Hg of diastolic blood pressure in average, our country could reduce 487000 strokes and 144000 coronary heart diseases every year, and save several billions of money for country.

At present, the treatment of hypertension mainly depends on the oral administration of Chinese and Western medicine. Western medicine of antihypertensive drugs have up to one hundred kinds and dominate the market of antihypertensive drugs, which decrease the blood pressure mainly by reducing blood volume and expanding blood vessels and its advantage is characterized in reducing the blood pressure quickly. However, there are also obvious side effects, such as hypokalemia, hypercholesterolemia, sexual dysfunction, irritating cough, angioneurotic edema and so on, these side effects all limit the long-term use for some patients. Compared to Western medicine, Chinese medicine has dialectical diagnosis for treatment of hypertension, which not only emphasizes the heterogeneity of hypertension and individual treatment, but also adjusts the body's yin and yang, qi to improve the systemic symptoms, furthermore, Chinese medicine has relatively small side effects and could be used for long-term, which is the unique advantage compared to Western medicine.

Sunflower leaf is dry leaf of Compositae sunflower, Helianthus annuus L., which is recorded on page 96 in "Chinese Materia Medica Standards of Shandong Province", 2002, as follows: Sunflower leaf is tasteless, bitter, smooth for nature and non-toxic, it can enter liver and stomach. Moreover, it can calm the liver, suppress the sthenic Yang and be helpful for digestion of food and make stomach healthy. Sunflower leaf can be used for treating hypertension, headache and dizziness. In ancient folk China, there was a record for using sunflower leaf to treatment of hypertension: for example, 30g sunflower leaves (fresh leaves for 60g) was boiled for thick soup, patients each took once at every night and morning, continuously for 7 days, insisting on taking had a good antihypertensive effect. Very few reports are about sunflower leaf as Chinese medicine for clinical application, its application for reducing blood pressure was reported in "Heilongjiang Medicine Journal" (Vol. 8, Issue 1, 1995, page 57) wherein "Kui pan jiang ya pian" made of sunflower head exhibited antihypertensive effect, but the preparation in this reference is only tablet using the crude extract of sunflower head or the compounds as the raw material, and this preparation is never seen in clinical use and for sale.

Sunflower leaf is widely distributed throughout our country, foreign study of sunflower leaf's chemical composition and chemical and biological activity are reported greatly. Especially, some new compounds from sunflower leaf with biological activity are reported in recent years, but for other activities of sunflower leaf, head and stem, especially the antihypertensive activity which has been widely used in folk, the study is almost blank.

In Chinese patent ZL200310105398.7 with the title of "Hypertension treating traditional Chinese medicine preparation and its preparing process", it was disclosed that sunflower leaf was applied to the antihypertensive compound of traditional Chinese medicine to reduce blood pressure. However, so far, there is no report about the application for treating hypertension using active ingredients or efficient component from the extract of sunflower leaf, head and stem, and also no report for using active ingredients or effective parts from the extract of sunflower leaf, head and stem as a medicinal and edible new resource to develop market, prepare food or functional food in large-scale, especially food or functional food for hypertension patents.

### Description of Invention

In order to better understand the present invention, some terms are defined below. The terms defined in this invention have the common meanings with the person skilled in the relative filed.

Unless specified otherwise, the term "the extract of total flavonoids" is the extract through extraction, separation and purification of leaves, heads or stems of sunflower with the content of total flavonoids more than 50%.

Unless specified otherwise, the term "the extract of total terpenes" is the extract through extraction, separation and purification of leaves, heads or stems of sunflower with the content of total terpenes more than 50%.

Unless specified otherwise, the term "the extract of total organic acids" is the extract through extraction, separation and purification of leaves, heads or stems of sunflower with the content of total organic acids more than 50%.

According to one aspect of this invention, it provides a pharmaceutical composition containing the extract of sunflower, which comprises the extract of total flavonoids from leaves, heads or stems of sunflower, in which the content of total flavonoids is more than 50 wt.%. And in the pharmaceutical composition, the content of the extract of total flavonoids is 30-70 wt.%.

Wherein, the extract of total flavonoids mainly contains nevadensin (5,7-dihydroxy-6,8,4'-trimethoxyflavone), sudachitin (5,7,4'-trihydroxy-6,8,3'- trimethoxyflavone), sudachitin demethoxy (5,7,4'-trihydroxy-6,8-dimethoxyflavone), hymenoxin (5,7-dihydroxy-6,8,3',4'-tetramethoxyflavone), hispidulin (5,7,4'- trihydroxy-6-methoxyflavone), acerosin (5,7,3'-trihydroxy-6,8,4'- trimethoxyflavone), 7,4'-dihydroxyflavone and so on; Preferably, the content of nevadensin in the extract of total flavonoids is 0.5-60 wt.%; More preferably, the content of nevadensin in the extract of total flavonoids is 20-60 wt.%.

Wherein, the pharmaceutical composition also comprises one or two kinds of the extract of total terpenes and the extract of total organic acids from leaves, heads or stems of sunflower; the extract of total terpenes mainly contains sesquiterpenoid and sesquiterpene lactone, for example, Guaianolide, Garmacranolide, Heliangolide and Edudesmane; diterpene and diterpene acid such as turpentine alkyl and kaurane; and triterpene such as oleanolic saponin and rearranged turpentine (rearranged 3,4-seco-tirueallane-type triterpenoids); the extract of total organic acids mainly contains chlorogenic acid, citric acid, malic acid and fumaric acid, preferably, the content of chlorogenic acid in the extract of total organic acids is 10-60 wt.%. In the said pharmaceutical composition, the content of the extract of total terpenes is 10-40 wt.% and the content of the extract of total organic acids is 10-30 wt.%.

Wherein, the extract of total flavonoids is prepared as the following steps:
(1) Leaves, heads or stems of sunflower are extracted with water or ethanol solution (10-95%, v/v) as the solvent, the extract solution is concentrated to 0.5-2 g/ml pharmaceutical liquor;
(2) The pharmaceutical liquor in step (1) is passed through macroporous adsorption resin column, such as ADS-17 type, ADS-F8 type or LS-300 type, eluting with water, and then conducting gradient elution with 5-95% (v/v) ethanol solution,
   preferably with 10%-70% (v/v) ethanol solution, collecting ethanol eluent, concentrating, drying to obtain the extract of total flavonoids.

The extract of total terpenes is prepared as the following steps:
(1) Leaves, heads or stems of sunflower are extracted with water or ethanol solution (10-95%, v/v) as the solvent, the extract solution is concentrated to 0.5-2 g/ml pharmaceutical liquor;
(2) The pharmaceutical liquor in step (1) is extracted by organic solvent such as chloroform, combining the extract liquor, concentrating, and dissolving in water, and passing through polyamide column, processing gradient elution with 5-95% (v/v) ethanol solution, preferably with 10%-70% (v/v) ethanol solution, collecting eluent, concentrating, and drying to obtain the extract of total terpenes.

Wherein, the extract of total organic acids is prepared as the following steps:
(1) Leaves, heads or stems of sunflower are extracted with water or 10-95% (v/v) ethanol solution as the solvent, the extract solution is concentrated to 0.5-2 g/ml pharmaceutical liquor;
(2) The pharmaceutical liquor in step (1) is passed through macroporous adsorption resin column, such as ADS-17 type, ADS-F8 type, AB-8 type or LS-300 type, eluting with water, combining effluent of macroporous adsorbing resin and water eluent, passing through anion exchange resin, such as 201X7 strongly basic styrene type anion exchange resin, eluting with ammoniacal ethanol, collecting eluent, concentrating, and drying to obtain the extract of total organic acids.

According to another aspect of this invention, it provides a method to produce the above pharmaceutical composition, which comprises the step of extracting the extract of total flavonoids from sunflower, wherein the step of extracting the extract of total flavonoids from sunflower is as follows:
Leaves, heads or stems of sunflower are extracted with water or ethanol solution as the solvent, the extract solution is concentrated and then passed through macroporous adsorption resin column, eluting with water, then conducting gradient elution with ethanol solution, collecting ethanol eluent, concentrating, drying to obtain the extract of total flavonoids.

Preferably, the said method also comprises the steps of extracting the extract of total terpenes and/or total organic acids from sunflower, wherein the step of extracting the extract of total terpenes is as follows:
Leaves, heads or stems of sunflower are extracted with water or ethanol solution as the solvent, the extract solution is concentrated and then extracted by organic solvent, combining the extract liquor, concentrating, and dissolving in water, and passing through polyamide column, processing gradient elution with ethanol solution, collecting eluent, concentrating, and drying to obtain the extract of total terpenes.

The step of extracting the extract of total organic acids from sunflower is as follows:
Leaves, heads or stems of sunflower are extracted with water or ethanol solution as the solvent, the extract solution is concentrated and then passed through macroporous adsorption resin column, eluting with water, combining effluent of macroporous adsorbing resin and water eluent, passing through anion exchange resin, eluting with ammoniacal ethanol, collecting eluent, concentrating, and drying to obtain the extract of total organic acids.

According to another aspect of this invention, it provides a pharmaceutical preparation, which comprises the above pharmaceutical composition and pharmaceutically-acceptable carriers and/or excipients, preferably, the formulations of the pharmaceutical preparation contain tablet, capsule, granules, pill, oral solution, syrup, injection, slow-release formulation, controlled-release formulation, targeted delivery and other pharmaceutically-acceptable formulations; wherein the tablet comprises oral tablet, lozenge, dispersible tablet, effervescent tablet, gel tablet, slow-release tablet and controlled-release tablet; wherein the injection comprises small injection, large injection, powder injection and emulsion.

According to another aspect of this invention, it provides the use of the above pharmaceutical composition or pharmaceutical preparation for preparing medicament in preventing and treating hypertension diseases and for preparing food and functional food in auxiliary reducing blood pressure.

According to another aspect of this invention, it provides a method for preventing and treating hypertension diseases, which comprises the step of administering the above pharmaceutical composition or pharmaceutical preparation to the individual.

Also described herein is a health-care food therapy method suitable for hypertension diseases, which comprises the step of feeding the above functional food containing pharmaceutical composition or pharmaceutical preparation to the individual.

This invention is described in detail in the following:
This invention provides a pharmaceutical composition containing the extract from leaves of sunflower, the pharmaceutical composition contains the extract of total flavonoids from leaves, heads or stems of sunflower, the content of total flavonoids is more than 50 wt. %, and wherein the content of total flavonoids in the pharmaceutical composition is 30-70 wt. %. This pharmaceutical composition could also comprise one or two kinds of the extract of total terpenes and the extract of total organic acids from leaves of sunflower.

For the pharmaceutical composition containing the extract from leaves of sunflower in this invention, the raw material in preparing the extract could also be selected from heads or stems of sunflower.
Various pharmaceutically-acceptable formulations are produced through adding various pharmaceutically-acceptable adjuvants to the pharmaceutical composition containing the extract from leaves of sunflower in this invention. The formulations comprise ordinary tablet (common tablet, enteric-coated tablet, film-coated tablet, sugar-coated tablet, extract tablet, dispersible tablet, scratch tablet, chewable tablet, lozenge, sublingual tablet and oral effervescent tablet), ordinary capsule (hard capsule, soft capsule, pill capsule and enteric-coated capsule); slow and/or controlled release formulation, including slow-release tablet, slow-release coated tablet, controlled-release tablet (including rapid-release tablet), slow-release capsule, controlled-release capsule; oral liquid formulation, including oral solution, oral suspension, oral emulsion, oral glue formulation, oral solution, emulsion liquid, emulsion formulation, colloidal solution, mixture, tincture, drops, suspension drops, decoction formulation; pill, including pellets and drop pill; granule, including sugar granule, sugar-free granule, enteric-coated granule, granule for oral suspension, and oral powder; injection, including liquid injection and powder injection.

The pharmaceutical composition in this invention comprises the extract of total flavonoids from leaves of sunflower, which is prepared according to the following technical solution:
(1) Leaves of sunflower is extracted with water or ethanol solution (10-95%, v/v) as the solvent, the extract solution is concentrated to 0.5-2 g/ml pharmaceutical liquor;
(2) The pharmaceutical liquor is passed through macroporous adsorption resin column, eluting with water, and then conducting gradient elution with 5%-95% (v/v) ethanol solution, collecting ethanol eluent, concentrating, drying to obtain the extract of total flavonoids.

In the extraction of total flavonoids from leaves of sunflower, 10%-70% (v/v) ethanol solution is used in conducting gradient elution on macroporous resin.

In the preparation of the extract of total flavonoids from leaves of sunflower in the pharmaceutical composition, the content of total flavonoids in sunflower leaf is 0.5-25%.

The extract of total flavonoids from sunflower leaf in the pharmaceutical composition mainly comprises nevadensin (5,7-dihydroxy-6,8,4'-trimethoxyflavone), sudachitin (5,7,4'-trihydroxy-6,8,3'-trimethoxyflavone), sudachitin demethoxy (5,7,4'-trihydroxy-6,8-dimethoxyflavone), hymenoxin (5,7-dihydroxy-6,8,3',4'-tetramethoxyflavone), hispidulin (5,7,4'-trihydroxy-6-methoxyflavone), acerosin (5,7,3'-trihydroxy-6,8,4'-trimethoxyflavone), 7,4'-dihydroxyflavone and other more than 10 kinds of flavonoids, wherein the content of nevadensin (5,7-dihydroxy-6,8,4'-trimethoxyflavone) in the extract of total flavonoids is 20-60 wt.%.

The above flavonoids have the following structural formula (I):

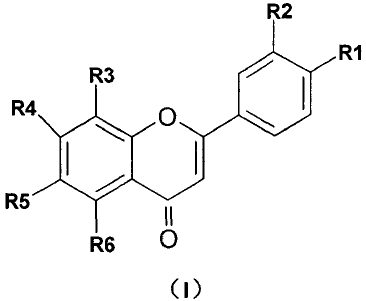

Wherein, R1 represents OMe, OH;
R2 represents H, OMe, OH;
R3 represents OMe, H;
R4 represents OH;
R5 represents OMe, H;
R6 represents OH, H;
When R1=OMe, R2=H, R3=OMe, R4=OH, R5=OMe, R6=OH, formula (I) represents nevadensin (5,7-dihydroxy-6,8,4'-trimethoxyflavone);
When R1=OH, R2=OMe, R3=OMe, R4=OH, R5=OMe, R6=OH, formula (I) represents sudachitin (5,7,4'-trihydroxy-6,8,3'-trimethoxyflavone);
When R1=OH, R2=H, R3=OMe, R4=OH, R5=OMe, R6=OH, formula (I) represents sudachitin demethoxy (5,7,4'-trihydroxy-6,8-dimethoxyflavone);
When R1=OMe, R2=OMe, R3=OMe, R4=OH, R5=OMe, R6=OH, formula (I) represents hymenoxin (5,7-dihydroxy-6,8,3',4'-tetramethoxyflavone);
When R1=OH, R2=H, R3=H, R4=OH, R5=OMe, R6=OH, formula (I) represents hispidulin (5,7,4'-trihydroxy-6-methoxyflavone);
When R1=OMe, R2=OH, R3=OMe, R4=OH, R5=OMe, R6=OH, formula (I) represents acerosin (5,7,3'-trihydroxy-6,8,4'-trimethoxyflavone);
When R1=OH, R2=H, R3=H, R4=OH, R5=H, R6=H, formula (I) represents 7,4'-dihydroxyflavone.

Wherein the content of nevadensin (5,7-dihydroxy-6,8,4'-trimethoxyflavone) in the extract of total flavonoids is 0.5-60 wt.%.

The pharmaceutical composition in this invention could also contain one or two kinds of the extract of total terpenes and the extract of total organic acids from leaves of sunflower, wherein the extract of total terpenes from leaves of sunflower is prepared as follows: Leaves of sunflower are extracted with water or ethanol solution (10-95%, v/v) as the solvent, the extract solution is concentrated to 0.5-2 g/ml pharmaceutical liquor, the pharmaceutical liquor is extracted by organic solvent, combining the extract liquor, concentrating, and dissolving in water, and passing through polyamide column, processing gradient elution with 5-95% (v/v) ethanol solution, collecting eluent, concentrating, and drying to obtain the extract of total terpenes. The extract of total organic acids from leaves of sunflower is prepared as the following steps: combining effluent of macroporous adsorbing resin in the step (2) of preparing the extract of total flavonoids and water eluent, passing through anion exchange resin, eluting with ammoniacal ethanol, collecting eluent, concentrating, and drying to obtain the extract of total organic acids.

In the extraction of total terpenes from leaves of sunflower, concentrating the extract liquor and passing through polyamide column, 10%-70% (v/v) ethanol solution is used in conducting gradient elution.

In the preparation of the extract of total terpenes from leaves of sunflower, the content of total terpenes in sunflower leaf is 3-20 wt.%.

The extract of total terpenes from sunflower leaf in the pharmaceutical composition mainly comprises sesquiterpenoid and sesquiterpene lactone, for example, Guaianolide, Garmacranolide, Heliangolide and Edudesmane; diterpene and diterpene acid such as turpentine alkyl and kaurane; and triterpene such as oleanolic saponin and rearranged turpentine (rearranged 3,4-seco-tirueallane-type triterpenoids).

The pharmaceutical composition in this invention could also comprises the extract of total organic acids from leaves of sunflower. In the preparation of the extract of total organic acids from leaves of sunflower, the content of total organic acids in sunflower leaf is 2%-25 wt.%.

The extract of total organic acids from sunflower leaf in the pharmaceutical composition mainly comprises chlorogenic acid, citric acid, malic acid and fumaric acid, wherein the content of chlorogenic acid in the extract of total organic acids is 10-60 wt.%.

The use of the pharmaceutical composition containing the extract of sunflower for preparing medicament in preventing and treating hypertension disease is also provided in this invention.

The use of the pharmaceutical composition containing the extract of sunflower for preparing food and functional food in auxiliary reducing blood pressure is also provided in this invention.

In clinical, the dosage of the pharmaceutical composition containing the extract of sunflower in this invention could be 0.3~0.8g/ time and 3 times a day.

The extract of sunflower leaf with anti-hypertensive activity which has the efficient total flavonoids component with the content of total flavonoids more than 50% is explored, researched and separated under the guide of biological activity, the preparation is easy, low-cost and suitable for industrial large-scale process. Furthermore, the extract of total terpenes from sunflower leaf with the content more than 50% in the efficient total terpenes component and the extract of total organic acids with the content more than 50% in the efficient total organic acids component are separated in this invention, adding one or two kinds of the above two extracts to the extract of total flavonoids, efficient anti-hypertensive effect is also exhibited by the obtained pharmaceutical composition. According to the experiment, sunflower head and stem also contain similar flavonoids, terpenes and organic acids to that of sunflower leaf, and their components in the extract after extraction, separation and determination are basically same with the components in the extract of sunflower leaf. Therefore, for the composition containing the extract from leaves of sunflower in this invention, the raw material in preparing the extract could also be selected from heads or stems of sunflower. The pharmaceutical composition containing the extract from leaves of sunflower in this invention can be used to prepare Chinese medicine for preventing and treating hypertension disease and food or functional food in auxiliary reducing blood pressure with controlled quality, efficient effect and good safety.

Proved by the test of blood pressure effect on renovascular hypertensive rats, systolic and diastolic blood pressure is decreased significantly (P < 0.05) after administering the pharmaceutical composition containing the extract from leaves of sunflower in this invention. This result prompts that the pharmaceutical composition containing the extract from leaves of sunflower could be used as the active ingredient in preparing medicament for preventing and treating hypertension disease and in preparing food or functional food in auxiliary reducing blood pressure, and so it's pharmaceutical and edible use is realized.

### Description of Figures

Figure 1 shows the preparation process of extracting total flavonoids and total organic acids from leaves of sunflower according to embodiment of this invention.
Figure 2 shows the preparation process of extracting total terpenes from leaves of sunflower according to embodiment of this invention.

### Preferable Embodiments of Invention

Further explanation is elaborated combined with the following embodiments, but it will be understood that the embodiments are only for explaining this invention and the scope of invention is not limited by the embodiments.

It will be understood that for the method, process, device, apparatus, materials, etc. which are mentioned but not elaborated in this invention, the person skilled in the art could access using well known method, process, device, apparatus, materials, etc. in this art or conventional knowledge and technology in this art.

In the following, the embodiments about the preparation method for the pharmaceutical composition containing the extract from leaves of sunflower and biological activity of the pharmaceutical composition are explained in detail.

### Example 1: preparation of the extract of total flavonoids from sunflower leaf

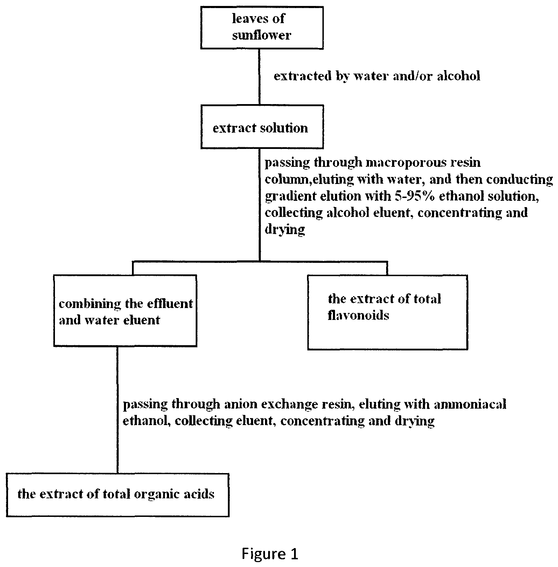

According to the flow chart as represented in figure 1, 20kg sunflower leaf and 12 times of 80% ethanol solution were added to extraction tank, the sunflower leaf was extracted twice and 1 hour every time, the extract solution was combined, filtered and concentrated to 1g/ml pharmaceutical liquor. The pharmaceutical liquor was passed through ADS-17 type macroporous adsorption resin column (Produced by Anhui Sanxing Resin Technology Company Limited), eluting with 3 bv water, and then eluting with 3 bv 10% ethanol solution and 3 bv 30% ethanol solution, collecting the ethanol eluent, concentrating and drying to obtain the extract of total flavonoids of 0.32kg.

Determinated by UV spectrophotometry, the content of total flavonoids in this extract of total flavonoids is 52.2%, the total flavonoids comprise nevadensin (5,7-dihydroxy-6,8,4'-trimethoxyflavone), sudachitin (5,7,4'-trihydroxy-6,8,3'-trimethoxyflavone), sudachitin demethoxy (5,7,4'-trihydroxy-6,8-dimethoxyflavone), hymenoxin (5,7-dihydroxy -6,8,3',4'- tetramethoxyflavone), hispidulin (5,7,4'-trihydroxy- 6- methoxyflavone), acerosin (5,7,3'-trihydroxy-6,8,4'- trimethoxyflavone), 7,4'-dihydroxyflavone and other more than 10 kinds of flavonoids, wherein the content of nevadensin (5,7-dihydroxy-6,8,4'-trimethoxyflavone) in the extract of total flavonoids is 1.8 wt.%.

### Example 2: preparation of the extract of total flavonoids from sunflower leaf

According to the flow chart as represented in figure 1, 20kg sunflower leaf and 12 times of 50% ethanol solution were added to extraction tank, the sunflower leaf was extracted for two hours and the residue was extracted for 1 hour after adding another 10 times of 50% ethanol solution, the extract solution was combined, filtered and concentrated to 0.8g/ml pharmaceutical liquor. The pharmaceutical liquor was passed through ADS-F8 type macroporous adsorption resin column (Produced by Chemical Factory of Nankai University), eluting with 3 bv water, and then eluting with 3 bv 10% ethanol solution and 3 bv 70% ethanol solution, collecting the ethanol eluent, concentrating and drying to obtain the extract of total flavonoids of 0.29kg.

Determinated by UV spectrophotometry, the content of total flavonoids in this extract of total flavonoids is 63.2%, the total flavonoids comprise nevadensin (5,7-dihydroxy-6,8,4'- trimethoxyflavone), sudachitin (5,7,4'-trihydroxy-6,8,3'-trimethoxyflavone), sudachitin demethoxy (5,7,4'-trihydroxy-6,8-dimethoxyflavone), hymenoxin (5,7-dihydroxy-6,8,3',4'-tetramethoxyflavone), hispidulin (5,7,4'-trihydroxy-6- methoxyflavone), acerosin (5,7,3'-trihydroxy-6,8,4'- trimethoxyflavone), 7,4'-dihydroxyflavone and other more than 10 kinds of flavonoids, wherein the content of nevadensin (5,7-dihydroxy-6,8,4'- trimethoxyflavone) in the extract of total flavonoids is 28.6 wt.%.

### Example 3: preparation of the extract of total flavonoids from sunflower head

According to the flow chart as represented in figure 1, 20kg sunflower head and 10 times of 50% ethanol solution were added to extraction tank, the sunflower head was extracted for two hours and the residue was extracted 1 hour after adding another 10 times of 50% ethanol solution, the extract solution was combined, filtered and concentrated to 0.8g/ml pharmaceutical liquor. The pharmaceutical liquor was passed through LS-300 type macroporous adsorption resin column (Produced by Xi'an Lanshen Resin Limited Company), eluting with 3 bv water, and then eluting with 3 bv 10% ethanol solution and 3 bv 70% ethanol solution, collecting the eluent of 70% ethanol solution, concentrating and drying to obtain the extract of total flavonoids of 0.21kg.

Determinated by UV spectrophotometry, the content of total flavonoids in this extract of total flavonoids is 58.4%, the total flavonoids comprise nevadensin (5,7-dihydroxy-6,8,4'-trimethoxyflavone), sudachitin (5,7,4'-trihydroxy-6,8,3'-trimethoxyflavone), sudachitin demethoxy (5,7,4'-trihydroxy- 6,8-dimethoxyflavone), hymenoxin (5,7-dihydroxy-6,8,3',4'-tetramethoxyflavone), hispidulin (5,7,4'-trihydroxy-6-methoxyflavone), acerosin (5,7,3'-trihydroxy- 6,8,4'-trimethoxyflavone), 7,4'-dihydroxyflavone and other more than 10 kinds of flavonoids, wherein the content of nevadensin (5,7-dihydroxy-6,8,4'- trimethoxyflavone) in the extract of total flavonoids is 19.6 wt.%.

### Example 4: preparation of the extract of total flavonoids from sunflower stem

According to the flow chart as represented in figure 1, 20kg sunflower stem and 10 times of 90% ethanol solution were added to extraction tank, the sunflower stem was extracted for two hours and the residue was extracted 1 hour after adding another 10 times of 50% ethanol solution, the extract solution was combined, filtered and concentrated to 0.8g/ml pharmaceutical liquor. The pharmaceutical liquor was passed through LS-300 type macroporous adsorption resin column (Produced by Xi'an Lanshen Resin Limited Company), eluting with 3 bv water, and then eluting with 3 bv 10% ethanol solution and 3 bv 50% ethanol solution, collecting the eluent of 50% ethanol solution, concentrating and drying to obtain the extract of total flavonoids of 0.16kg.

Determinated by UV spectrophotometry, the content of total flavonoids in this extract of total flavonoids is 51.4%, the total flavonoids comprise nevadensin (5,7-dihydroxy-6,8,4'-trimethoxyflavone), sudachitin (5,7,4'-trihydroxy-6,8,3'-trimethoxyflavone), sudachitin demethoxy (5,7,4'-trihydroxy-6,8-dimethoxyflavone), hymenoxin (5,7-dihydroxy-6,8,3',4'-tetramethoxyflavone), hispidulin (5,7,4'-trihydroxy-6-methoxyflavone), acerosin (5,7,3'-trihydroxy-6,8,4'-trimethoxyflavone), 7,4'-dihydroxyflavone and other more than 10 kinds of flavonoids, wherein the content of nevadensin (5,7-dihydroxy-6,8,4'-trimethoxyflavone) in the extract of total flavonoids is 10.6 wt.%.

### Example 5: preparation of the extract of total terpenes from sunflower leaf

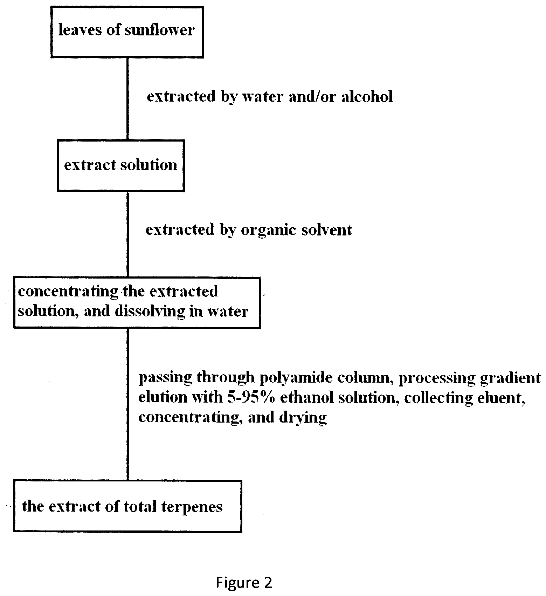

According to the flow chart as represented in figure 2, 40kg sunflower leaf was added to extraction tank, the sunflower leaf was extracted twice with 70% ethanol solution, the amount of 70% ethanol solution was 12 times per time and every time for extraction was for two hours. The extract solution was combined, filtered and concentrated to 1g/ml pharmaceutical liquor. The pharmaceutical liquor was extracted by same amount of chloroform for 5 times, combining the extract liquor, concentrating, and passing through polyamide column, eluting with 1 bv water, and then eluting with 3 bv 30% ethanol solution and 2 bv 70% ethanol solution, combining the eluents, concentrating and drying to obtain the extract of total terpenes of 0.51kg.

Determinated by UV spectrophotometry, the content of total terpenes in this extract of total terpenes is 51.4%.

### Example 6: preparation of the extract of total terpenes from sunflower stem

According to the flow chart as represented in figure 2, 25kg sunflower stem was added to extraction tank, the sunflower stem was extracted twice with 30% ethanol solution, the amount of 30% ethanol solution was 12 times per time and every time for extraction was for two hours. The extract solution was combined, filtered and concentrated to 1g/ml pharmaceutical liquor. The pharmaceutical liquor was extracted by same amount of chloroform for 5 times, combining the extract liquor, concentrating, and passing through polyamide column, eluting with 1 bv water, and then eluting with 3 bv 30% ethanol solution and 2 bv 70% ethanol solution, combining the eluents, concentrating and drying to obtain the extract of total terpenes of 0.21kg.

Determinated by UV spectrophotometry, the content of total terpenes in this extract of total terpenes is 51.4%.

### Example 7: preparation of the extract of total organic acids from sunflower leaf

50kg sunflower leaf was added to extraction tank, the sunflower leaf was extracted twice with 30% ethanol solution, the amount of 30% ethanol solution was 10 times per time and every time for extraction was for two hours. The extract solution was combined, filtered and concentrated to 1g/ml pharmaceutical liquor. The pharmaceutical liquor was passed through AB-8 type macroporous adsorption resin column (Produced by Anhui Sanxing Resin Technology Company Limited), eluting with 3 bv water, combining effluent of macroporous adsorbing resin and water eluent, passing through 201X7 strongly basic styrene type anion exchange resin (Produced by Beijing Research Institute of Chemical Engineering and Metallurgy), eluting with 4 bv 20% ammoniacal ethanol, collecting eluent, concentrating, and drying to obtain the extract of total organic acids of 0.62kg.

Determinated by UV spectrophotometry, the content of total organic acids in this extract of total organic acids is 61.7%.

### Example 8: preparation of the extract of total organic acids from sunflower head

30kg sunflower head was added to extraction tank, the sunflower head was extracted twice with 80% ethanol solution, the amount of 80% ethanol solution was 10 times per time and every time for extraction was for two hours. The extract solution was combined, filtered and concentrated to 1g/ml pharmaceutical liquor. The pharmaceutical liquor was passed through AB-8 type macroporous adsorption resin column (Produced by Anhui Sanxing Resin Technology Company Limited), eluting with 3 bv water, combining effluent of macroporous adsorbing resin and water eluent, passing through 201X7 strongly basic styrene type anion exchange resin (Produced by Beijing Research Institute of Chemical Engineering and Metallurgy), eluting with 4 bv 20% ammoniacal ethanol, collecting eluent, concentrating, and drying to obtain the extract of total organic acids of 0.42kg.

Determinated by UV spectrophotometry, the content of total organic acids in this extract of total organic acids is 61.7%.

### Example 9: determination of the content of total flavonoids in the extract of total flavonoids from sunflower leaf

The steps of using UV spectrophotometry to determine the content of total flavonoids in the extract of total flavonoids in this invention are as follows:
1, Preparation of the control solution
   2 mg nevadensin as the control sample was exactly taken and put into 10ml volumetric flask, adding methanol for dissolving the sample to the mark and shaking.
2, Preparation of standard curve
   0.1ml, 0.2ml, 0.3ml, 0.4ml, 0.5ml and 0.6ml solution of control sample were exactly taken and put into 25ml volumetric flask respectively, exactly adding 5ml 1% Aluminum chloride aqueous solution respectively and shaking, then exactly adding 8ml 1mol/L potassium acetate aqueous solution and shaking, adding water to the mark and shaking. Using related reagents as blank, the above samples were measured absorbance at 228 nm of wavelength according to the UV - visible spectrophotometry (Chinese Pharmacopoeia, one appendix V A , 2005), then the standard curve was made using absorbance for the vertical and concentration as the abscissa.
3, Determination
   3 mg test power in this invention was exactly taken and put into 10ml volumetric flask, adding 8ml 70% ethanol solution, treating with ultrasonic wave for 30 min (100W, 40kHz), adding 70% ethanol solution to the mark after cooling, shaking and filtering. 1.0 ml filtered solution was exactly taken and put into 25ml volumetric flask and exactly adding 5ml 1% Aluminum chloride aqueous solution respectively and shaking, then exactly adding 8ml 1mol/L sodium acetate aqueous solution and shaking, adding water to the mark and shaking. Using related reagents as blank, the above sample was measured absorbance at 228 nm of wavelength according to the UV - visible spectrophotometry (Chinese Pharmacopoeia, one appendix V A , 2005), then the amount of nevadensin in the test sample was read from the standard curve, calculating to obtain that 1g sample contains 620mg total flavonoids based on nevadensin.

### Example 10: determination of the content of nevadensin in the extract of total flavonoids from sunflower leaf

The steps of using High Performance Liquid Chromatography to determine the content of nevadensin in the extract of total flavonoids in this invention are as follows:
1, Experiment of Chromatographic conditions and system suitability
   Octadecyl bonded silica is vector, acetonitrile-0.4% ammonium citrate (40:60) is mobile phase and the detection wavelength is 284nm.
2, Preparation of the solution of control sample
   The right amount of nevadensin as the control sample was exactly taken, adding methanol to get the solution with the concentration of 50µg/ml.
3, Preparation of the solution of test sample
   15 mg test power in this invention was exactly taken and put into 10ml volumetric flask, adding 8ml 70% ethanol solution, treating with ultrasonic wave for 30 min (100W, 40kHz), adding 70% ethanol solution to the mark after cooling, shaking and filtering.
4, Determination
   10µl solution of control sample and 10µl solution of test sample were taken exactly and respectively, the solution was injected into the liquid chromatograph and determined. 20mg nevadensin was contained in 1g test power of this invention.

### Example 11: determination of the content of total terpenes in the extract of total terpenes from sunflower leaf

The steps of using UV spectrophotometry to determine the content of total terpenes in the extract of total terpenes in this invention are as follows:
1, Preparation of the solution of control sample
   2 mg panaxadiol as the control sample was exactly taken and put into 5ml volumetric flask, adding methanol for dissolving the sample to the mark and shaking.
2, Preparation of standard curve
   0.1ml, 0.2ml, 0.3ml, 0.4ml, 0.5ml and 0.6ml solution of control sample were exactly taken and put into 25ml volumetric flask respectively, exactly adding 0.2ml 5% fresh acetic acid solution of vanillin and 0.8ml perchloric acid respectively and shaking, heating at 70°C for 15min, shaking, and then adding acetic acid to the mark and shaking. Using related reagents as blank, the above samples were measured absorbance at 550 nm of wavelength according to the UV - visible spectrophotometry (Chinese Pharmacopoeia, one appendix V A , 2005), then the standard curve was made using absorbance for the vertical and concentration as the abscissa.
3, Determination
   3 mg test power in this invention was exactly taken and put into 10ml volumetric flask, adding 8ml 70% ethanol solution, treating with ultrasonic wave for 30 min (100W, 40kHz), adding 70% ethanol solution to the mark after cooling, shaking and filtering. 1.0 ml filtered solution was exactly taken and put into 25ml volumetric flask and exactly adding 0.2ml 5% fresh acetic acid solution of vanillin and 0.8ml perchloric acid respectively and shaking, heating at 70°C for 15min, shaking, and then adding acetic acid to the mark and shaking. Using related reagents as blank, the above sample was measured absorbance at 550 nm of wavelength according to the UV - visible spectrophotometry (Chinese Pharmacopoeia, one appendix V A , 2005), then the amount of panaxadiol in the test sample was read from the standard curve, calculating to obtain that 1g sample contains 560mg total terpenes based on panaxadiol.

### Example 12: determination of the content of total organic acids in the extract of total organic acids from sunflower leaf

The steps of using UV spectrophotometry to determine the content of total organic acids in the extract of total organic acids in this invention are as follows:
1, Preparation of the solution of control sample
   2 mg chlorogenic acid as the control sample was exactly taken and put into 5ml volumetric flask, adding methanol for dissolving the sample to the mark and shaking.
2, Preparation of standard curve
   0.1ml, 0.2ml, 0.3ml, 0.4ml, 0.5ml and 0.6ml solution of control sample were exactly taken and put into 25ml volumetric flask respectively, exactly adding 0.8ml fresh phloroglucinol solution respectively and shaking, heating at boiling water bath for 40min, shaking, and then adding acetic acid to the mark after cooling, shaking. Using related reagents as blank, the above samples were measured absorbance at 443 nm of wavelength according to the UV - visible spectrophotometry (Chinese Pharmacopoeia, one appendix V A , 2005), then the standard curve was made using absorbance for the vertical and concentration as the abscissa.
3, Determination
   3 mg test power in this invention was exactly taken and put into 10ml volumetric flask, adding 8ml 70% ethanol solution, treating with ultrasonic wave for 30 min (100W, 40kHz), adding 70% ethanol solution to the mark after cooling, shaking and filtering. 1.0 ml filtered solution was exactly taken and put into 25ml volumetric flask and exactly adding 0.8ml fresh phloroglucinol solution respectively and shaking, heating at boiling water bath for 40min, shaking, and then adding acetic acid to the mark after cooling, shaking. Using related reagents as blank, the above sample was measured absorbance at 443 nm of wavelength according to the UV - visible spectrophotometry (Chinese Pharmacopoeia, one appendix V A , 2005), then the amount of chlorogenic acid in the test sample was read from the standard curve, calculating to obtain that 1g sample contains 560mg total organic acids based on chlorogenic acid.

### Example 13: The anti hypertensive effect of the pharmaceutical composition containing the extract from sunflower leaf for renovascular hypertensive rats

Proved by the test of antihypertensive effect on renovascular hypertensive rats, the pharmaceutical composition containing the extract from sunflower leaf exhibits antihypertensive activity.
1, Test material
   Antihypertensive sample 1 (the extract of total flavonoids from sunflower leaf, wherein the content of total flavonoids from sunflower leaf is 63.2%, dosage for person is 0.25g / person / day), tan powder.
   Antihypertensive sample 2 (the mixture of the extract of total flavonoids and the extract of total organic acids from sunflower leaf (1 : 0.8), wherein dosage for person is 0.35g / person / day), dark brown powder.
   Antihypertensive sample 3 (the mixture of the extract of total flavonoids and the extract of total terpenes from sunflower leaf (1 : 0.6), wherein dosage for person is 0.40g / person / day), dark brown powder.
   Antihypertensive sample 4 (the mixture of the extract of total flavonoids, the extract of total terpenes and the extract of total organic acids from sunflower leaf (1 : 0.8 : 0.6), wherein dosage for person is 0.5g / person / day), dark brown powder.
   Positive control sample: Compound Kendir Leaves I (6 pills / day for person), 100 pills / bottle.
2, Experimental animals and equipment
   25 renovascular hypertensive rats, clean grade, weighing 357 ± 42g, provided by Shandong Lukang Pharmaceutical Co., Ltd., License Number: SCXK Lu 20080002.
   Major equipment: Non-invasive tail artery blood pressure measurement and analysis system, ALC-NIBP type, produced by Shanghai Alcott Biotech. Co., Ltd.
3, Test methods
   25 renovascular hypertensive rats were divided into 5 groups randomly, that is a group of antihypertensive sample 1, 100 mg / kg, a group of antihypertensive sample 2, 150 mg / kg, a group of antihypertensive sample 3, 50 mg / kg, a group of antihypertensive sample 4, 200 mg / kg, a group of positive control Compound Kendir Leaves I, 3 pills / kg, every group had 5 rats. The blood pressure of rats as before drug value was determined by Non-invasive tail artery blood pressure measurement and analysis system before administering medicine. Then one-time intragastric administrating, each treatment group was given medical liquid with different concentration for 2ml / 200g of rat. The change of rat tail artery blood pressure was determined by Non-invasive tail artery blood pressure measurement and analysis system after 2.0 hours of administering medicine.
4, Statistics and results
   Experimental data were presented as mean ± standard deviation (X ± s). Before and after of their own were compared using t-test for statistical analysis.
5, The results were summarized in Table 1.

**Table 1 Antihypertensive effect on renovascular hypertensive rats through one-time intragastric administration of antihypertensive samples (X ± s)**

| group | dosage (mg/kg) | Before administering medicine | | | 2.0 h after administering medicine | | |
|---|---|---|---|---|---|---|---|
| | | Systolic blood pressure (mmHg) | Diastolic blood pressure (mmHg) | Average blood pressure (mmHg) | Systolic blood pressure (mmHg) | Diastolic blood pressure (mmHg) | Average blood pressure (mmHg) |
| Antihypertensive sample 1 | 100 | 159.52± 37.89 | 127.14± 33.76 | 138.07± 34.92 | 127.59± 31.58 | 93.62±2 6.04* | 103.87±28.57* |
| Antihypertensive sample 2 | 150 | 148.32± 28.07 | 117.55± 28.78 | 128.60± 29.37 | 128.44± 33.37* | 89.81±1 7.21* | 102.21±21.56* |
| Antihypertensive sample 3 | 50 | 156.52± 33.89 | 120.14± 31.96 | 132.07± 32.72 | 124.59± 30.29 | 91.62±2 5.68* | 105.87±27.69* |
| Antihypertensive sample 4 | 200 | 144.32± 28.07 | 116.59± 26.78 | 125.43± 24.37 | 123.44± 32.47* | 88.41±1 9.21* | 101.21±20.56* |
| Compound Kendir Leaves I | 3 pills | 142.70± 25.98 | 109.66± 22.60 | 120.58± 24.08 | 128.32± 15.61 | 94.05±1 5.03 | 105.20±14.81 |

Compared to that of before administering medicine, *P<0.05.

The results showed that antihypertensive sample 1 and 3 exhibited evidently antihypertensive activity for the diastolic blood pressure and average arterial pressure of renovascular hypertensive rats, although no significant effect for systolic blood pressure was on statistics, it had a reduced trend. Antihypertensive samples 2 and 4 both exhibited evidently antihypertensive activity for blood pressure of renovascular hypertensive rats.

### Example 14: Preparation of tablets of pharmaceutical composition containing the extract from sunflower leaf

The tablets of this invention are prepared as follows, 205g extract of total flavonoids from sunflower leaf in example 1 is taken, added 95g starch, mixed, granulated, sifted and added 2g microcrystalline cellulose, 0.5g magnesium stearate, mixed and pressed into 1000 pills.

### Example 15: Preparation of granules of pharmaceutical composition containing the extract from sunflower leaf

The granules of this invention are prepared as follows, 405g extract of total flavonoids and total organic acids from sunflower leaf in example 1 and 2 is taken, added 200g lactose, 400g dextrin, pelleting, granulated, sifted and made into 1000g granules.

### Example 16: Test of clinical observation for the patients with liver yang hyperactive and essential hypertension using tablets containing the extract of total flavonoids from sunflower leaf

### 1, General Information

46 patients were all from the Department of Cardiology clinic, they were randomly divided into experimental group and control group. Experimental group had 26 patients, wherein 12 males and 14 females, the average age was 55.14 ± 11.45, the mean disease duration was 10.87 ± 7.13 years; control group had 20 patients, wherein 11 males and 9 females, the average age was 55.97 ± 10.85, the mean disease duration was 11.46 ± 6.98 years. There were no significant differences for the two groups in gender, age, disease duration, pre-treatment blood pressure values and comparisons of Chinese medicine for individual symptoms, etc. (P>0.05)

### 2, Treating method

The experimental group was given tablets of the extract of total flavonoids from sunflower leaf, 2 pills every time and 3 times per day; the control group was given tablets of Compound Kendir Leaves, 4 pills every time and 3 times per day. Four weeks was a course of treatment for both two groups, the blood pressure and treating effect were gathered into statistics after one course of treatment.

### 3, Evaluation standard

Evaluation standard of antihypertensive effect: Referring to the standard formulated in the forum of Chinese cardiovascular epidemiology and crowd prevention, 1979; Evaluation standard of TCM symptom: Referring to the regulations in «New Drugs of Traditional Chinese Drug Guidelines for Clinical Research» promulgated by the Ministry of Health, 2002.

### 4, Statistitics

The analysis of Ridit tests t.

### 5, Treatment results

(1) The antihypertensive effect and extent of the two groups were summarized in Tables 2-4.

**Table 2 Comparison of antihypertensive effect**

| | n | Markedly effective | Effective | No effect | Total effective rate |
|---|---|---|---|---|---|
| Experimental group | 26 | 10 | 12 | 4 | 84.6% |
| Control group | 20 | 5 | 8 | 7 | 65% |

By the analysis of Ridit, P<0.05, the result of experimental group was better than that of control group.

**Table 3 Comparison of the reduced extent of systolic blood pressure (kPa)**

| | Pre-treatment | After treatment |
|---|---|---|
| Experimental group | 22.9±2.6 | 18.7±2.1*# |
| Control group | 22.8±2.5 | 22.3±2.2 |

| | | |
|---|---|---|
| *meaned P<0.01 compared to that of pre-treatment, # meaned P<0.01 compared to that of control group. | | |

**Table 4 Comparison of the reduced extent of diastolic blood pressure (kPa)**

| | Pre-treatment | After treatment |
|---|---|---|
| Experimental group | 13.8±1.2 | 11.2±1.0*# |
| Control group | 13.9±0.3 | 12.1±1.2 |

| | | |
|---|---|---|
| *meaned P<0.01 compared to that of pre-treatment, # meaned P<0.01 compared to that of control group. | | |

According to Table 2, 10 cases were markedly effective and 12 cases were effective in the 26 cases of experimental group, the total effective rate was 84.6%, which was better than that of control group, by the analysis of Ridit, there was significant difference between two groups' effect (P<0.05). According to Table 3 and 4, systolic and diastolic blood pressure of two groups' pre and after treatment with self-comparison respectively showed a significant difference (P<0.01), and a significant difference (P<0.01) also existed between that of experimental group and control group after treatment.
(2) The efficacy of TCM symptoms:
The result of experimental group in improving the traditional Chinese medicine for individual symptoms such as dizziness, headache, dizziness and stretching, spasm of nape, flushed face and congested eyes, palpitation, chest tightness, chest pain, insomnia and tinnitus was better than that of control group, irritability and blurred vision were excepted. The significant difference (P<0.05 or P<0.01) existed between that of the two groups. The results showed that tablets containing the extract of total flavonoids from sunflower leaf in this invention not only exhibited efficient antihypertensive activity, but also improved the symptoms significantly; and no side effect was observed during the treatment.

## Claims

1. A pharmaceutical composition containing sunflower extract, which composition comprises an extract of total flavonoids from the leaves, heads or stems of sunflower, in which the content of total flavonoids is more than 50 wt.%, and wherein the content of the extract of total flavonoids in the pharmaceutical composition is 30-70 wt. %.

2. A pharmaceutical composition according to claim 1, wherein the extract of total flavonoids contains nevadensin (5,7-dihydroxy-6,8,4'-trimethoxyflavone), sudachitin (5,7,4'-trihydroxy-6,8,3'-trimethoxyflavone), sudachitin demethoxy (5,7,4'-trihydroxy-6,8-dimethoxyflavone), hymenoxin (5,7-dihydroxy-6,8,3',4'-tetramethoxyflavone), hispidulin (5,7,4'-trihydroxy-6-methoxyflavone), acerosin (5,7,3'-trihydroxy-6,8,4'-trimethoxyflavone), 7,4'-dihydroxyflavone, preferably, the content of nevadensin in the extract of total flavonoids is 0.5-60 wt.%.; more preferably, the content of nevadensin in the extract of total flavonoids is 20-60 wt.%.

3. A pharmaceutical composition according to claim 1 or 2, wherein the pharmaceutical composition also comprises:
an extract of total terpenes, wherein the content of total terpenes is more than 50 wt. %; and/or
an extract of total organic acids, wherein the content of total organic acids is more than 50 wt. % from the leaves, heads or stems of sunflower;
wherein the extract of total terpenes contains sesquiterpenoid and sesquiterpene lactone, for example, Guaianolide, Germacranolide, Heliangolide and Eudesmane; diterpene and diterpene acid such as turpentine alkyl and kaurane; and triterpene such as oleanolic saponin and rearranged turpentine (rearranged 3,4-seco-tirueallane-type triterpenoids) and wherein the extract of total organic acids contains chlorogenic acid, citric acid, malic acid and fumaric acid, preferably, the content of chlorogenic acid in the extract of total organic acids is 10-60 wt.%.

4. A pharmaceutical composition according to any one of claims 1 to 3, wherein the extract of total flavonoids is prepared by the following steps:
(1) the leaves, heads or stems of sunflower are extracted with water or ethanol solution (10-95%, v/v) as the solvent, and the extract solution is concentrated to 0.5-2 g/ml pharmaceutical liquor;
(2) the pharmaceutical liquor obtained in step (1) is then passed through a macroporous adsorption resin column, such as ADS-17 type, ADS-F8 type or LS-300 type, and eluted with water, a gradient elution with 5-95% (v/v) ethanol solution is then conducted, preferably with 10%-70% (v/v) ethanol solution, and the resulting ethanol eluent is collected, concentrated, and dried to obtain the extract of total flavonoids.

5. A pharmaceutical composition according to claim 3, wherein the extract of total terpenes is prepared by the following steps:
(1) the leaves, heads or stems of sunflower are extracted with ethanol solution (10-95%, v/v) as the solvent, and the extract solution is concentrated to 0.5-2 g/ml pharmaceutical liquor;
(2) the pharmaceutical liquor obtained in step (1) is extracted by an organic solvent such as chloroform, the extract liquor is combined, concentrated, and dissolved in water, and then passed through a polyamide column, using gradient elution with 5-95% (v/v) ethanol solution, preferably with 10%-70% (v/v) ethanol solution, the resulting eluent is collected, concentrated, and dried to obtain the extract of total terpenes.

6. A pharmaceutical composition according to claim 3, wherein the extract of total organic acids is prepared by the following steps:
(1) the leaves, heads or stems of sunflower are extracted with water or 10-95% (v/v) ethanol solution as the solvent, and the extract solution is concentrated to 0.5-2 g/ml pharmaceutical liquor;
(2) the pharmaceutical liquor obtained in step (1) is passed through a macroporous adsorption resin column, such as ADS-17 type, ADS-F8 type, AB-8 type or LS-300 type, and eluted with water, the combined effluent from the macroporous adsorbing resin and water elution is then passed through an anion exchange resin (such as 201X7 strongly basic styrene type anion exchange resin), and eluted with ammoniacal ethanol, the resulting eluent is collected, concentrated, and dried to obtain the extract of total organic acids.

7. A method for preparing a pharmaceutical composition of any one of claims 1 to 6, which comprises the steps of extracting the extract of total flavonoids from sunflower, wherein the steps for extracting the extract of total flavonoids from sunflower are as follows:
the leaves, heads or stems of sunflower are extracted with water or ethanol solution as the solvent, the extract solution is concentrated and then passed through a macroporous adsorption resin column, and eluted with water, a gradient elution with ethanol solution is then conducted, the resultingethanol eluent is collected, concentrated, and dried to obtain the extract of total flavonoids, preferably, the method also comprises the steps of extracting the extract of total terpenes and/or total organic acids from sunflower, wherein the steps for extracting the extract of total terpenes are as follows:
the leaves, heads or stems of sunflower are extracted with ethanol solution as the solvent, the extract solution is concentrated and then extracted by organic solvent, the extracted liquors are combined, concentrated, and dissolved in water, and passed through a polyamide column, using gradient elution with ethanol solution, the resulting eluent is collected, concentrated, and dried to obtain the extract of total terpenes,
and wherein the steps of extracting the extract of total organic acids from sunflower are as follows:
the leaves, heads or stems of sunflower are extracted with water or ethanol solution as the solvent, the extract solution is concentrated and then passed through macroporous adsorption resin column, eluted with water, the combined effluent of the macroporous adsorbing resin and water eluent is then passed through an anion exchange resin, and eluted with ammoniacal ethanol, the resulting eluent is collected, concentrated, and dried to obtain the extract of total organic acids.

8. A pharmaceutical preparation comprising a pharmaceutical composition of any one of claims 1 to 6 and a pharmaceutically-acceptable carrier and/or excipient, preferably, wherein the pharmaceutical preparation is in the form of an ordinary tablet (common tablet, enteric-coated tablet, film-coated tablet, sugar-coated tablet, extract tablet, dispersible tablet, scratch tablet, chewable tablet, lozenge, sublingual tablet and oral effervescent tablet), ordinary capsule (hard capsule, soft capsule, pill capsule and enteric-coated capsule); slow and/or controlled release formulation, including slow-release tablet, slow-release coated tablet, controlled-release tablet (including rapid-release tablet), slow-release capsule and controlled-release capsule; oral liquid formulation, including oral solution, oral suspension, oral emulsion, oral glue formulation, oral solution, emulsion liquid, emulsion formulation, colloidal solution, mixture, tincture, drops, suspension drops and decoction formulation; pill, including pellets and drop pill; granule, including sugar granule, sugar-free granule, enteric-coated granule, granule for oral suspension, and oral powder; or injection, including liquid injection and powder injection.

9. Use of the pharmaceutical composition of any one of claims 1 to 6 or the pharmaceutical preparation of claim 8 in the manufacture of a medicament for preventing and treating hypertension disease or for the preparation of food and functional food for the auxiliary reduction of blood pressure.

10. A pharmaceutical composition of any one of claims 1 to 6 or a pharmaceutical preparation of claim 8 for use in preventing or treating hypertension disease.

11. A pharmaceutical composition of any one of claims 1 to 6 or a pharmaceutical preparation of claim 8 for use in the auxiliary reduction in blood pressure, wherein the pharmaceutical composition or preparation is in a food or functional food.

## Patentansprüche

1. Pharmazeutische Zusammensetzung, die einen Sonnenblumenextrakt enthält, wobei die Zusammensetzung einen Extrakt aus Gesamtflavonoiden von den Blättern, den Köpfen oder den Stängeln einer Sonnenblume umfasst, wobei der Gehalt von Gesamtflavonoiden mehr als 50 Gew.-% beträgt und wobei der Gehalt des Extrakts aus Gesamtflavonoiden in der pharmazeutischen Zusammensetzung 30-70 Gew.-% beträgt.

2. Pharmazeutische Zusammensetzung nach Anspruch 1, wobei der Extrakt aus Gesamtflavonoiden Nevadensin (5,7-Dihydroxy-6,8,4'-trimethoxyflavon), Sudachitin (5,7,4'-Trihydroxy-6,8,3'-trimethoxyflavon), Sudachitindemethoxy (5,7,4'-Trihydroxy-6,8-dimethoxyflavon), Hymenoxin (5,7-Dihydroxy-6,8,3',4'-tetramethoxyflavon), Hispidulin (5,7,4'-Trihydroxy-6-methoxyflavon), Acerosin (5,7,3'-Trihydroxy-6,8,4'-trimethoxyflavon), 7,4'-Dihydroxyflavon enthält, vorzugsweise wobei der Gehalt von Nevadensin im Extrakt aus Gesamtflavonoiden 0,5-60 Gew.-% beträgt; stärker bevorzugt wobei der Gehalt von Nevadensin im Extrakt aus Gesamtflavonoiden 20-60 Gew.-% beträgt.

3. Pharmazeutische Zusammensetzung nach Anspruch 1 oder 2, wobei die pharmazeutische Zusammensetzung ferner Folgendes umfasst:
einen Extrakt aus Gesamtterpenen, wobei der Gehalt von Gesamtterpenen mehr als 50 Gew.-% beträgt; und/oder
einen Extrakt aus gesamten organischen Säuren, wobei der Gehalt von gesamten organischen Säuren mehr als 50 Gew.-% von den Blättern, den Köpfen oder den Stängeln einer Sonnenblume beträgt;
wobei der Extrakt aus Gesamtterpenen Folgendes enthält: Sesquiterpenoid und Sesquiterpenlacton, beispielsweise Guaianolid, Germacranolid, Heliangolid und Eudesman; Diterpen und Diterpensäure, wie etwa Terpentinalkyl und Kauran; und Triterpen, wie etwa Oleanolsaponin und umgelagertes Terpentin (umgelagerte Triterpenoide vom Typ 3,4-Secotirueallan), und wobei der Extrakt aus gesamten organischen Säuren Chlorogensäure,
Citronensäure, Hydroxybernsteinsäure und Fumarsäure enthält, vorzugsweise wobei der Gehalt von Chlorogensäure im Extrakt aus gesamten organischen Säuren 10-60 Gew.-% beträgt.

4. Pharmazeutische Zusammensetzung nach einem der Ansprüche 1 bis 3, wobei der Extrakt aus Gesamtflavonoiden durch die folgenden Schritte hergestellt wird:
(1) die Blätter, die Köpfe oder die Stängel einer Sonnenblume werden mit Wasser oder Ethanollösung (10-95 Vol.-%) als das Lösungsmittel extrahiert und die Exktraktlösung wird auf 0,5-2 g/ml pharmazeutischen Liquor konzentriert;
(2) der in Schritt (1) gewonnene pharmazeutische Liquor wird dann durch eine makroporöse Adsorptionsharzsäule, wie etwa vom Typ ADS-17, vom Typ ADS-F8 oder vom Typ LS-300, durchgeleitet,
und mit Wasser eluiert, eine Gradientenelution mit 5-95 Vol.-% Ethanollösung wird dann durchgeführt, vorzugsweise mit 10-70 Vol.-% Ethanollösung, und der resultierende Ethanoleluent wird gesammelt, konzentriert und getrocknet, um den Extrakt aus Gesamtflavonoiden zu gewinnen.

5. Pharmazeutische Zusammensetzung nach Anspruch 3, wobei der Extrakt aus Gesamtterpenen durch die folgenden Schritte hergestellt wird:
(1) die Blätter, die Köpfe oder die Stängel einer Sonnenblume werden mit Ethanollösung (10-95 Vol.-%) als das Lösungsmittel extrahiert und die Exktraktlösung wird auf 0,5-2 g/ml pharmazeutischen Liquor konzentriert;
(2) der in Schritt (1) gewonnene pharmazeutische Liquor wird durch ein organisches Lösungsmittel, wie etwa Chloroform, extrahiert, der Extraktliquor wird kombiniert, konzentriert und in Wasser gelöst, und dann durch eine Polyimidsäule durchgeleitet, unter Verwendung einer Gradientenelution mit 5-95 Vol.-% Ethanollösung, vorzugsweise mit 10-70 Vol-.% Ethanollösung, wobei der resultierende Eluent gesammelt, konzentriert und getrocknet wird, um den Extrakt aus Gesamtterpenen zu gewinnen.

6. Pharmazeutische Zusammensetzung nach Anspruch 3, wobei der Extrakt aus gesamten organischen Säuren durch die folgenden Schritte hergestellt wird:
(1) die Blätter, die Köpfe oder die Stängel einer Sonnenblume werden mit Wasser oder 10-95 Vol.-% Ethanollösung als das Lösungsmittel extrahiert und die Exktraktlösung wird auf 0,5-2 g/ml pharmazeutischen Liquor konzentriert;
(2) der in Schritt (1) gewonnene pharmazeutische Liquor wird durch eine makroporöse Adsorptionsharzsäule, wie etwa vom Typ ADS-17, vom Typ ADS-F8, vom Typ AB-8 oder vom Typ LS-300, durchgeleitet und mit Wasser eluiert, das kombinierte Ablaufmaterial von der makroporösen adsorbierenden Harz- und Wasserelution wird dann durch ein Anionenaustauschharz (wie etwa 201X7 stark basischem Anionenaustauschharz vom Styroltyp) geleitet und mit ammoniakalischem Ethanol eluiert, wobei der resultierende Eluent gesammelt, konzentriert und getrocknet wird, um den Extrakt aus gesamten organischen Säuren zu gewinnen.

7. Verfahren zum Herstellen einer pharmazeutischen Zusammensetzung nach einem der Ansprüche 1 bis 6, das die Schritte des Extrahierens des Extrakts aus Gesamtflavonoiden aus einer Sonnenblume umfasst, wobei die Schritte zum Extrahieren des Extrakts aus Gesamtflavonoiden aus einer Sonnenblume wie folgt sind:
die Blätter, die Köpfe oder die Stängel einer Sonnenblume werden mit Wasser oder Ethanollösung als das Lösungsmittel extrahiert, die Extraktlösung wird konzentriert und dann durch eine makroporöse Adsorptionsharzsäule durchgeleitet und mit Wasser eluiert, eine Gradientenelution mit Ethanollösung wird dann durchgeführt, der resultierende Ethanoleluent wird gesammelt, konzentriert und getrocknet, um den Extrakt aus Gesamtflavonoiden zu gewinnen, wobei das Verfahren vorzugsweise ferner die Schritte des Extrahierens des Extrakts aus Gesamtterpenen und/oder gesamten organischen Säuren aus einer Sonnenblume umfasst, wobei die Schritte zum Extrahieren des Extrakts aus Gesamtterpenen wie folgt sind:
die Blätter, die Köpfe oder die Stängel einer Sonnenblume werden mit Ethanollösung als das Lösungsmittel extrahiert, die Extraktlösung wird konzentriert und dann durch organisches Lösungsmittel extrahiert, die extrahierten Liquores werden kombiniert, konzentriert und in Wasser gelöst und durch eine Polyimidsäule durchgeleitet, unter Verwendung einer Gradientenelution mit Ethanollösung, wobei der resultierende Eluent gesammelt,
konzentriert und getrocknet wird, um den Extrakt aus Gesamtterpenen zu gewinnen, und wobei die Schritte des Extrahierens des Extrakts aus gesamten organischen Säuren aus einer Sonnenblume wie folgt sind:
die Blätter, die Köpfe oder die Stängel einer Sonnenblume werden mit Wasser oder Ethanollösung als das Lösungsmittel extrahiert, die Extraktlösung wird konzentriert und dann durch die makroporöse Adsorptionsharzsäule durchgeleitet, mit Wasser eluiert, das kombinierte Ablaufmaterial des makroporösen adsorbierenden Harz- und Wassereluenten wird dann durch ein Anionenaustauschharz durchgeleitet und mit ammoniakalischem Ethanol eluiert, der resultierende Eluent wird gesammelt, konzentriert und getrocknet, um den Extrakt aus gesamten organischen Säuren zu gewinnen.

8. Pharmazeutisches Präparat, umfassend eine pharmazeutische Zusammensetzung nach einem der Ansprüche 1 bis 6 und einen pharmazeutisch unbedenklichen Träger und/oder Hilfsstoff, vorzugsweise wobei das pharmazeutische Präparat in der Form einer gewöhnlichen Tablette (übliche Tablette, magensaftresistente Tablette, Filmtablette, Dragee, Extrakttablette, dispergierbare Tablette, Kratztablette, Kautablette, Lutschtablette, sublinguale Tablette und orale Brausetablette), einer gewöhnlichen Kapsel (Hartkapsel, Weichkapsel, Pillenkapsel und magensaftresistente Kapsel); einer Formulierung mit langsamer und/oder kontrollierter Freisetzung, einschließlich einer Tablette mit langsamer Freisetzung, einer Filmtablette mit langsamer Freisetzung, einer Tablette mit kontrollierter Freisetzung (einschließlich Tablette mit rascher Freisetzung), einer Kapsel mit langsamer Freisetzung und einer Kapsel mit kontrollierter Freisetzung; einer oralen Flüssigformulierung, einschließlich einer oralen Lösung, einer oralen Suspension, einer oralen Emulsion, einer oralen Klebstoffformulierung, einer oralen Lösung, einer Emulsionsflüssigkeit, einer Emulsionsformulierung, einer kolloidalen Lösung, eines Gemischs, einer Tinktur, von Tropfen, Suspensionstropfen und einer Dekoktformulierung; einer Pille, einschließlich Pellets und einer Tropfenpille; eines Granulats, einschließlich eines Zuckergranulats, eines zuckerfreien Granulats, eines magenresistenten Granulats, eines Granulats für orale Suspension und eines oralen Pulvers; oder einer Spritze, einschließlich einer Flüssiginjektion und einer Pulverinjektion, vorliegt.

9. Verwendung der pharmazeutischen Zusammensetzung nach einem der Ansprüche 1 bis 6 oder des pharmazeutischen Präparats nach Anspruch 8 beim Herstellen eines Arzneimittels zum Vorbeugen und Behandeln einer Hypertonieerkrankung oder zum Herstellen eines Nahrungsmittels und eines funktionellen Nahrungsmittels zum unterstützenden Senken von Blutdruck.

10. Pharmazeutische Zusammensetzung nach einem der Ansprüche 1 bis 6 oder pharmazeutisches Präparat nach Anspruch 8 zur Verwendung beim Vorbeugen oder Behandeln einer Hypertonieerkrankung.

11. Pharmazeutische Zusammensetzung nach einem der Ansprüche 1 bis 6 oder pharmazeutisches Präparat nach Anspruch 8 zur Verwendung beim unterstützenden Senken von Blutdruck, wobei die/das pharmazeutische Zusammensetzung oder Präparat in einem Nahrungsmittel oder funktionellen Nahrungsmittel vorliegt.

## Revendications

1. Composition pharmaceutique contenant un extrait de tournesol, dont la composition comprend un extrait de flavonoïdes totaux provenant des feuilles, des têtes ou des tiges de tournesol, la teneur en flavonoïdes totaux étant supérieure à 50 % en poids, et dans laquelle la teneur en extrait de flavonoïdes totaux dans la composition pharmaceutique est comprise entre 30 et 70 % en poids.

2. Composition pharmaceutique selon la revendication 1, dans laquelle l'extrait de flavonoïdes totaux contient de la névadensine (5,7-dihydroxy-6,8,4'-triméthoxyflavone), de la sudachitine (5,7,4'-trihydroxy-6,8,3'-triméthoxyflavone), de la sudachitine déméthoxy (5,7,4'-trihydroxy-6,8-diméthoxyflavone), de l'hyménoxine (5,7-dihydroxy-6,8,3',4'-tétraméthoxyflavone), de l'hispiduline (5,7,4'-trihydroxy-6-méthoxyflavone), de l'acérosine (5,7,3'-trihydroxy-6,8,4'-timéthoxyflavone), de la 7,4'-dihydroxyflavone, de préférence, la teneur en névadensine dans l'extrait de flavonoïdes totaux est comprise entre 0,5 et 60 % en poids ; de manière plus préférable, la teneur en névadensine dans l'extrait de flavonoïdes totaux est comprise entre 20 et 60 % en poids.

3. Composition pharmaceutique selon la revendication 1 ou 2, dans laquelle la composition pharmaceutique comprend également :
un extrait de terpènes totaux, dans laquelle la teneur en terpènes totaux est supérieure à 50 % en poids ; et/ou
un extrait d'acides organiques totaux provenant des feuilles, des têtes ou des tiges du tournesol, la teneur en acides organiques totaux étant supérieure à 50 % en poids ;
dans laquelle l'extrait de terpènes totaux contient du sesquiterpénoïde et du sesquiterpène lactone, par exemple, du guaianolide, du germacranolide, de l'héliangolide et de l'éudesmane ; du diterpène et de l'acide diterpénique comme de l'alkyle de térébenthine et du kaurane ; et du triterpène comme la saponine oléanolique et la térébenthine réarrangée (triterpénoïdes de type 3,4-séco-tiruéallane réarrangés) et dans laquelle l'extrait d'acides organiques totaux contient de l'acide chlorogénique, de l'acide citrique, de l'acide malique et de l'acide fumarique, de préférence, la teneur en acide chlorogénique dans l'extrait d'acides organiques totaux est comprise entre 10 et 60 % en poids.

4. Composition pharmaceutique selon l'une quelconque des revendications 1 à 3, dans laquelle l'extrait de flavonoïdes totaux est préparé selon les étapes suivantes :
(1) les feuilles, les têtes ou les tiges de tournesol sont extraites avec de l'eau ou une solution d'éthanol (10 à 95 %, v/v) comme solvant, et la solution d'extrait est concentrée jusqu'à une teneur en liqueur pharmaceutique de 0,5 à 2 g/ml ;
(2) la liqueur pharmaceutique obtenue à l'étape (1) est ensuite passée à travers une colonne de résine d'adsorption macroporeuse, comme le type ADS-17, le type ADS-F8 ou le type LS-300,
puis éluée avec de l'eau, une élution de gradient avec une solution d'éthanol de 5 à 95 % (v/v) est ensuite réalisée, de préférence une solution d'éthanol de 10 % à 70 % (v/v), et l'éluant d'éthanol résultant est ensuite recueilli, concentré puis séché pour obtenir l'extrait de flavonoïdes totaux.

5. Composition pharmaceutique selon la revendication 3, dans laquelle l'extrait de terpènes totaux est préparé selon les étapes suivantes :
(1) les feuilles, les têtes ou les tiges de tournesol sont extraites avec une solution d'éthanol (10 à 95 %, v/v) comme solvant, et la solution d'extrait est concentrée jusqu'à une teneur en liqueur pharmaceutique de 0,5 à 2 g/ml ;
(2) la liqueur pharmaceutique obtenue à l'étape (1) est extraite par un solvant organique comme le chloroforme, la liqueur d'extrait est combinée, concentrée et dissoute dans de l'eau, puis passée à travers une colonne de polyamide, en utilisant une élution de gradient avec une solution d'éthanol comprise entre 5 et 95 % (v/v), de préférence avec une solution d'éthanol comprise entre 10 % et 70 % (v/v), l'éluant résultant étant recueilli, concentré et séché pour obtenir l'extrait de terpènes totaux.

6. Composition pharmaceutique selon la revendication 3, dans laquelle l'extrait d'acides organiques totaux est préparé selon les étapes suivantes :
(1) les feuilles, les têtes ou les tiges de tournesol sont extraites avec de l'eau ou une solution d'éthanol de 10 à 95 % (v/v) comme solvant, et la solution d'extrait est concentrée jusqu'à une teneur en liqueur pharmaceutique de 0,5 à 2 g/ml ;
(2) la liqueur pharmaceutique obtenue à l'étape (1) est passée à travers une colonne de résine d'adsorption macroporeuse, comme le type ADS-17, le type ADS-F8, le type AB-8 ou le type LS-300, et éluée avec de l'eau, l'effluent combiné de la résine adsorbante macroporeuse et de l'élution d'eau est ensuite passé à travers une résine échangeuse d'anions (comme la résine échangeuse d'anions de type styrène 201X7 fortement basique) et élué avec de l'éthanol ammoniacal, l'éluant résultant est recueilli, concentré, puis séché pour obtenir l'extrait d'acides organiques totaux.

7. Procédé de préparation d'une composition pharmaceutique selon l'une quelconque des revendications 1 à 6, qui comprend les étapes d'extraction de l'extrait de flavonoïdes totaux provenant de tournesol, dans lequel les étapes d'extraction de l'extrait de flavonoïdes totaux provenant de tournesol sont comme suit :
les feuilles, les têtes ou les tiges de tournesol sont extraites avec de l'eau ou une solution d'éthanol comme solvant, la solution d'extrait est concentrée puis passée à travers une colonne de résine d'adsorption macroporeuse et éluée avec de l'eau, une élution de gradient avec une solution d'éthanol est ensuite réalisée, l'éluant d'éthanol résultant est recueilli,
concentré et séché pour obtenir l'extrait de flavonoïdes totaux, de préférence, le procédé comprend également les étapes d'extraction de l'extrait de terpènes totaux et/ou d'acides organiques totaux provenant de tournesol, dans lequel les étapes d'extraction de l'extrait de terpènes totaux sont comme suit :
les feuilles, les têtes ou les tiges de tournesol sont extraites avec une solution d'éthanol comme solvant, la solution d'extrait est concentrée puis extraite par un solvant organique, les liqueurs extraites sont combinées, concentrées et dissoutes dans de l'eau, puis passées à travers une colonne de polyamide, en utilisant une élution de gradient avec une solution d'éthanol, l'éluant résultant est recueilli, concentré puis séché pour obtenir l'extrait de terpènes totaux, et dans lequel les étapes d'extraction de l'extrait d'acides organiques totaux provenant de tournesol sont comme suit :
les feuilles, les têtes ou les tiges de tournesol sont extraites avec de l'eau ou une solution d'éthanol comme solvant, la solution d'extrait est concentrée puis passée à travers une colonne de résine d'adsorption macroporeuse, puis éluée avec de l'eau, l'effluent combiné de la résine adsorbante macroporeuse et de l'éluant d'eau est ensuite passé à travers une résine échangeuse d'anions et élué avec de l'éthanol ammoniacal, l'éluant résultant est recueilli, concentré puis séché pour obtenir l'extrait d'acides organiques totaux.

8. Préparation pharmaceutique comprenant une composition pharmaceutique selon l'une quelconque des revendications 1 à 6 et un support et/ou un excipient pharmaceutiquement acceptable(s), de préférence, dans laquelle la préparation pharmaceutique est sous la forme d'un comprimé ordinaire (comprimé courant, comprimé à enrobage entérique, comprimé enrobé par un film, comprimé enrobé de sucre, comprimé d'extrait, comprimé dispersible, comprimé à gratter, comprimé à mâcher, pastille, comprimé sublingual et comprimé oral effervescent), d'une capsule ordinaire (capsule dure, capsule mole, pilule et capsule à enrobage entérique) ; d'une formulation à libération lente et/ou contrôlée, y compris un comprimé à libération lente, un comprimé enrobé à libération lente, un comprimé à libération contrôlée (y compris un comprimé à libération rapide), une capsule à libération lente et une capsule à libération contrôlée ; d'une formulation orale liquide, y compris une solution orale, une suspension orale, une émulsion orale, une formulation de colle orale, une solution orale, un liquide d'émulsion, une formulation d'émulsion, une solution colloïdale, un mélange, une teinture, des gouttes, des gouttes de suspension et une formulation de décoction ; d'une pilule, y compris des pastilles et un comprimé lingual ; d'un granulé, y compris un granulé de sucre, un granulé sans sucre, un granulé à enrobage entérique, un granulé pour suspension orale et une poudre orale ; ou d'une injection, y compris une injection liquide et une injection de poudre.

9. Utilisation de la composition pharmaceutique selon l'une quelconque des revendications 1 à 6 ou de la préparation pharmaceutique selon la revendication 8 dans la fabrication d'un médicament pour prévenir et traiter l'hypertension ou pour la préparation d'aliments et d'aliments fonctionnels pour la réduction auxiliaire de la pression artérielle.

10. Composition pharmaceutique selon l'une quelconque des revendications 1 à 6 ou préparation pharmaceutique selon la revendication 8 à utiliser dans la prévention ou le traitement de l'hypertension.

11. Composition pharmaceutique selon l'une quelconque des revendications 1 à 6 ou préparation pharmaceutique selon la revendication 8 à utiliser dans la réduction auxiliaire de la pression artérielle, dans laquelle la composition ou préparation pharmaceutique est présente dans un aliment ou un aliment fonctionnel.
